(19) Europäisches Patentamt
European Patent Office
Office européen des brevets

(11) Veröffentlichungsnummer: **0 391 311 B1**

(12) **EUROPÄISCHE PATENTSCHRIFT**

(45) Veröffentlichungstag der Patentschrift: **16.03.94**

(51) Int. Cl.$^5$: **C07D 405/06**, A01N 43/653, A01N 43/50, //C07D303/32

(21) Anmeldenummer: **90106288.5**

(22) Anmeldetag: **02.04.90**

# (54) 1-Alkoxy-1-azolylmethyloxirane, Verfahren zu ihrer Herstellung und ihre Verwendung als Pflanzenschutzmittel.

(30) Priorität: **06.04.89 DE 3911059**

(43) Veröffentlichungstag der Anmeldung: **10.10.90 Patentblatt 90/41**

(45) Bekanntmachung des Hinweises auf die Patenterteilung: **16.03.94 Patentblatt 94/11**

(84) Benannte Vertragsstaaten: **AT BE CH DE DK ES FR GB GR IT LI NL SE**

(56) Entgegenhaltungen: **EP-A- 0 199 168**

(73) Patentinhaber: **BASF Aktiengesellschaft**
**Carl-Bosch-Strasse 38**
**D-67063 Ludwigshafen(DE)**

(72) Erfinder: **Seele, Rainer, Dr.**
**Leiblstrasse 3**
**D-6701 Fussgoenheim(DE)**
Erfinder: **Kober, Reiner, Dr.**
**Im Schlittweg 20**
**D-6701 Fussgoenheim(DE)**
Erfinder: **Goetz, Norbert, Dr.**
**Schoefferstrasse 25**
**D-6520 Worms 1(DE)**
Erfinder: **Ammermann, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**

**EP 0 391 311 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Azolverbindungen, Verfahren zu ihrer Herstellung und diese enthaltende Fungizide sowie Verfahren zur Bekämpfung von Pilzen.

Es ist bekannt,
cis-2-(1,2,4-Triazol-1-yl-methyl)-2-(tert.-butyl)-3-(4-chlorphenyl)-1-oxiran (DE-A-3218130) als Fungizid zu verwenden. Die fungizide Wirkung ist jedoch in einigen Fällen unbefriedigend.

Es wurde nun gefunden, daß 1-Alkoxy-1-azolylmethyloxirane der Formel I

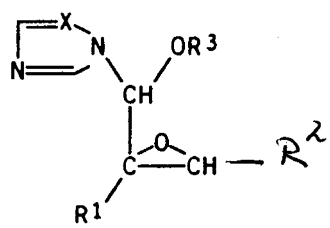

I,

in welcher

R[1] und R[2] gleich oder verschieden sind und jeweils $C_1$-$C_8$-Alkyl, $C_3$-$C_8$-Cycloalkyl, $C_5$-$C_8$-Cycloalkenyl, Tetrahydropyranyl, Norbornyl, Pyridyl, Naphthyl, Biphenyl oder Phenyl bedeutet, wobei diese Reste einfach bis dreifach durch Halogen, Nitro, Phenoxy, Amino, Alkyl, Alkoxy, oder Halogenalkyl mit jeweils 1 bis 4 C-Atomen substituiert sein können,

R[3] $C_1$-$C_4$-Alkyl bedeutet,

X den Rest CH oder N bedeutet,

sowie deren für Pflanzen verträgliche Säureadditionssalze und Metallkomplexe, eine bessere fungizide Wirkung besitzen als bekannte Azolverbindungen.

Die Verbindungen der Formel I enthalten asymmetrische C-Atome und können daher als Enantiomere und Diastereomere auftreten. Die Gemische von Diasteromeren lassen sich bei den erfindungsgemäßen Verbindungen in üblicher Weise, beispielsweise aufgrund ihrer unterschiedlichen Löslichkeit oder durch Säulenchromatographie trennen und in reiner Form isolieren. Die Racemate lassen sich bei den erfindungsgemäßen Verbindungen nach bekannten Methoden, beispielsweise durch Salzbildung mit einer optisch aktiven Säure, Trennung der diastereomeren Salze und Freisetzung der Enantiomeren mittels einer Base, trennen. Als fungizide Mittel kann man sowohl die einheitlichen Diastereomere bzw. Enantiomere als auch deren bei der Synthese anfallenden Gemische verwenden.

R[1] bzw. R[2] bedeuten beispielsweise $C_1$-$C_4$-Alkyl, Methyl, Ethyl, Isopropyl, n-Propyl, n-Butyl, sec-Butyl, tert.-Butyl, n-Pentyl, Neopentyl, 1-Naphthyl, 2-Naphthyl, p-Biphenyl, Phenyl, 2-Chlorphenyl, 2-Fluorphenyl, 2-Bromphenyl, 3-Chlorphenyl, 3-Bromphenyl, 3-Fluorphenyl, 4-Fluorphenyl, 4-Chlorphenyl, 4-Bromphenyl, 2,4-Dichlorphenyl, 2,3-Dichlorphenyl, 2,5-Dichlorphenyl, 2,6-Dichlorphenyl, 3-Chlor-6-fluorphenyl, 2-Methoxyphenyl, 3-Methoxyphenyl, 4-Methoxyphenyl, 2,4-Dimethoxyphenyl, 4-Ethylphenyl, 4-Isopropylphenyl, 4-tert.-Butylphenyl, 4-tert.-Butyloxyphenyl, 2-Chlor-4-fluorphenyl, 2-Chlor-6-fluorphenyl, 2-Chlor-6-methylphenyl, 3,4-Dimethoxyphenyl, 3-Phenoxyphenyl, 4-Phenoxyphenyl, 3-Nitrophenyl, 4-Nitrophenyl, 3-Aminophenyl, 4-Aminophenyl, 2-Aminophenyl, 2-Trifluormethylphenyl, 3-Trifluormethylphenyl, 4-Trifluormethylphenyl, 4-Phenylsulfonylphenyl, 3-Pyridyl, Tetrahydropyranyl, Cyclopropyl, Cyclopentyl, Cyclohexyl, 2-Cyclohexenyl, 3-Cyclohexenyl, Norbornyl.

Säureadditionssalze sind beispielsweise die Hydrochloride, Bromide, Sulfate, Nitrate, Phosphate, Oxalate oder Dodecylbenzolsulfonate. Die Wirksamkeit der Salze geht auf das Kation zurück, so daß es auf das Anion im allgemeinen nicht ankommt. Die erfindungsgemäßen Wirkstoffsalze werden hergestellt durch Umsetzung der 1-Alkoxy-1-azolylmethyloxirane (I) mit geeigneten Säuren.

Metallkomplexe der Wirkstoffe I oder ihrer Salze können mit Metallen wie Kupfer, Zink, Zinn, Mangan, Eisen, Kobalt oder Nickel gebildet werden, indem man die 1-Alkoxy-1-azolylmethyloxirane mit entsprechenden Salzen, z.B. den Chloriden oder Sulfaten wie Zinkchlorid, Kupfersulfat, Zinnchlorid, Mangansulfat, Eisensulfat umsetzt.

Die Verbindungen der Formel I können z. B. hergestellt werden, indem man eine Verbindung der Formel II

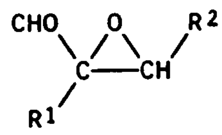

II,

in welcher $R^1$ und $R^2$ die angegebenen Bedeutungen haben, mit einer Verbindung der Formel III,

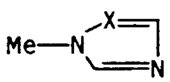

III,

in der Me ein Wasserstoffatom oder ein Metallatom (z.B. Na, K) und X CH oder N bedeuten, in Gegenwart von einem Thionylhalogenid und einem Alkohol der Formel IV

$R^3OH$ IV,

in der $R^3$ die oben angegebene Bedeutung hat, zur Umsetzung bringt.

Die Umsetzung erfolgt gegebenenfalls in Gegenwart eines Lösungs-oder Verdünnungsmittels im allgemeinen bei Temperaturen zwischen -30 und 80 °C. Zu den bevorzugten Lösungs- und Verdünnungsmitteln gehören Nitrile wie Acetonitril oder Propionitril, Ether wie Tetrahydrofuran, Diethylether, Dimethoxyethan, Dioxan oder Diisopropylether und insbesondere Kohlenwasserstoffe und Chlorkohlenwasserstoffe wie Pentan, Hexan, Toluol, Methylenchlorid, Chloroform, Tetrachlorkohlenstoff, Dichlorethan oder entsprechende Gemische.

Die neuen Ausgangsverbindungen II erhält man z.B. durch Epoxidierung der entsprechenden Olefine V,

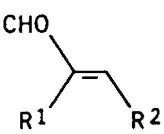

V,

in denen $R^1$ und $R^2$ die oben angegebenen Bedeutungen haben, mit Peroxycarbonsäuren wie Perbenzoesäure, 3-Chlorperbenzoesäure, 4-Nitroperbenzoesäure, Monoperphthalsäure, Peressigsäure, Perpropionsäure, Permaleinsäure, Monoperbernsteinsäure, Perpelargonsäure oder Trifluorperessigsäure in indifferenten Lösungsmitteln, vorzugsweise chlorierten Kohlenwasserstoffen, z. B. Methylenchlorid, Chloroform, Tetrachlorkohlenstoff, Dichlorethan, aber auch in organischen Säuren wie Essigsäure, in Estern wie Essigester, in Ketonen wie Aceton oder in Amiden wie Dimethylformamid, gegebenenfalls in Gegenwart eines Puffers wie Natriumacetat, Natriumcarbonat, Dinatriumhydrogenphosphat, Ethylendiamintetraessigsäure. Man arbeitet im allgemeinen bei Temperaturen von 10 bis 100 °C und katalysiert die Reaktion gegebenenfalls, z.B. mit Jod, Natriumwolframat oder Licht. Zur Oxidation eignen sich auch alkalische Lösungen von Wasserstoffperoxid (z.B. 20 bis 50 gew.%ig, vorzugsweise 25 bis 35 gew.%ig) in Alkoholen wie Methanol, Ethanol, Ketonen wie Aceton oder Nitrilen wie Acetonitril bei Temperaturen von im allgemeinen 10 bis 50 °C, vorzugsweise 20 bis 35 °C, insbesondere 25 bis 30 °C, sowie Alkylhydroperoxide, z. B. tert.-Butylhydroperoxid, unter Zusatz eines Katalysators, z. B. Natriumwolframat, Perwolframsäure, Molybdänhexacarbonyl oder Vanadylacetylacetonat. Die genannten Oxidationsmittel lassen sich z. T. in situ erzeugen.

Die Verbindungen V lassen sich entsprechend allgemein bekannter Verfahren zur Aldehydsynthese (vgl. z.B. Houben-Weyl-Müller, Methoden der Organischen Chemie, Georg Thieme Verlag, Stuttgart 1983, Bd E 3) herstellen.

Die folgenden Beispiele erläutern die Herstellung der Wirkstoffe.

I. Herstellung der Ausgangsstoffe

Beispiel A

Herstellung von E/Z-2-(4-Fluorphenyl)-3-phenyl-propenal

Zu einer Lösung von 26,5 g Benzaldehyd in 300 ml Methanol werden 4,2 g Natriumhydroxid in 30 ml Wasser gegeben. Das Reaktionsgemisch wird auf 10 ° C gekühlt und schnell 36 g 4-Fluorphenylacetaldehyd zugesetzt, wobei die Temperatur in der Lösung auf 30-40 ° C ansteigt. Nach 10stündigem Rühren bei 40 ° C werden die ausgefallenen Kristalle aus der abgekühlten Reaktionslösung abgesaugt. Man erhält 31,6 g (56 %) E/Z-2-(4-Fluorphenyl)-3-phenyl-propenal mit dem Schmelzpunkt 87-94 ° C.

Beispiel B

Herstellung von cis-2-Formyl-2-(4-fluorphenyl)-3-phenyl-oxiran

67,8 g E/Z-2-(4-Fluorphenyl)-3-phenyl-propenal werden in 300 ml Methanol gelöst und 1 ml konzentrierte Natronlauge zugesetzt. Die Reaktionslösung wird bei 0 ° C gerührt, während 20,5 g Wasserstoffperoxyd (ca. 50 gew. %ig) langsam zugetropft werden, wobei die Innentemperatur von 30 ° C nicht überschritten wird. Nach beendeter Zugabe wird sechs Stunden bei Raumtemperatur gerührt, anschließend 100 ml Wasser zugesetzt und die entstandene Emulsion mit Methyl-tert.-butylether ausgeschüttelt. Die isolierte organische Phase wird daraufhin über Natriumsulfat getrocknet und eingeengt. Man erhält 55,9 g (77 %) cis-2-Formyl-2-(4-fluorphenyl)3-phenyl-oxiran.

II. Herstellung der Endprodukte

Beispiel 1

Zu einer Lösung von 17,5 g Triazol in 75 ml Methylenchlorid werden bei 0 ° C unter Stickstoffatmosphäre 7,4 g Thionylchlorid gegeben. Nach beendeter Zugabe wird bei Raumtemperatur für 30 Minuten gerührt und anschließend 2 g Methanol und 10 g cis-2-Formyl-2-(4-fluorphenyl)-3-phenyl-oxiran zugesetzt. Nachdem das Reaktionsgemisch 12-15 Stunden bei Raumtemperatur rührte, wird der Lösung 100 ml Wasser zugesetzt und die organische Phase abgetrennt. Die verbleibende wäßrige Phase wird zweimal mit Methylenchlorid ausgeschüttelt und die gesammelten organischen Phasen zweimal mit gesättigter Natriumhydrogencarbonat-Lösung gewaschen. Die isolierte organische Phase wird daraufhin über Natriumsulfat getrocknet, eingeengt und der verbleibende Rückstand durch Flash-Chromatographie an Kieselgel (Essigester/n-Hexan = 9:1) gereinigt. Man erhält 2,3 g (18 %) 1'RS-cis-2-[1-(1,2,4-Triazol-1-yl)-1-methoxy-methyl]-2-(4-fluorphenyl)-3-phenyl-oxiran als 5:1-Diastereomerengemisch, Fp: 135-138 ° C.

Entsprechend Beispiel 1 können die in der Tabelle aufgeführten Verbindungen hergestellt werden.

Tabelle:

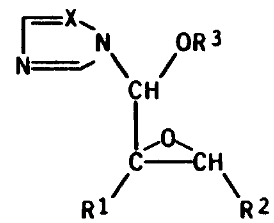

I

| Bsp. Nr. | $R^1$ | $R^2$ | $R^3$ | X | Schmp/IR | Isomer |
|---|---|---|---|---|---|---|
| 1 | 4-F-$C_6H_4$ | $C_6H_5$ | $CH_3$ | N | 135-138 °C | $D_1:D_2$=5:1 |
| 2 | 4-F-$C_6H_4$ | $C_6H_5$ | $CH_3$ | CH | Harz | $D_1:D_2$=4:1 |
| 3 | 4-F-$C_6H_4$ | $C_6H_5$ | $C_2H_5$ | N | | |
| 4 | 4-F-$C_6H_4$ | 2-Cl-$C_6H_4$ | $CH_3$ | N | | |
| 5 | 4-F-$C_6H_4$ | 2-Cl-$C_6H_4$ | $CH_3$ | CH | | |
| 6 | 4-F-$C_6H_4$ | 2-Cl-$C_6H_4$ | $C_2H_5$ | N | | |
| 7 | 4-F-$C_6H_4$ | 2-Cl-$C_6H_4$ | $C_3H_7$ | N | | |
| 8 | 4-F-$C_6H_4$ | 2-Cl-$C_6H_4$ | iso-$C_3H_7$ | N | | |
| 9 | 4-F-$C_6H_4$ | 2-Cl-$C_6H_4$ | $C_4H_9$ | N | | |
| 10 | 4-F-$C_6H_4$ | 3-Cl-$C_6H_4$ | $CH_3$ | N | | |
| 11 | 4-F-$C_6H_4$ | 4-Cl-$C_6H_4$ | $CH_3$ | N | | |

Tabelle: (Fortsetzung)

| Bsp. Nr. | $R^1$ | $R^2$ | $R^3$ | X | Schmp/IR | Isomer |
|---|---|---|---|---|---|---|
| 12 | 4-F-$C_6H_4$ | 4-Cl-$C_6H_4$ | $C_2H_5$ | N | | |
| 13 | 4-F-$C_6H_4$ | 2,4-$Cl_2$-$C_6H_3$ | $CH_3$ | N | | |
| 14 | 4-F-$C_6H_4$ | 2,4-$Cl_2$-$C_6H_3$ | $C_2H_5$ | N | | |
| 15 | 4-F-$C_6H_4$ | 2-Cl-4-F-$C_6H_3$ | $CH_3$ | N | | |
| 16 | 4-F-$C_6H_4$ | 2-$CF_3$-$C_6H_4$ | $CH_3$ | N | | |
| 17 | 4-F-$C_6H_4$ | 2-$CF_3$-$C_6H_4$ | $CH_3$ | CH | | |
| 18 | 4-F-$C_6H_4$ | 2-$CF_3$-$C_6H_4$ | $C_2H_5$ | N | | |
| 19 | 4-F-$C_6H_4$ | 4-$CF_3$-$C_6H_4$ | $CH_3$ | N | | |
| 20 | 4-F-$C_6H_4$ | 4-$CF_3$-$C_6H_4$ | $C_2H_5$ | N | | |
| 21 | 4-F-$C_6H_4$ | 2-F-$C_6H_4$ | $CH_3$ | N | | |
| 22 | 4-F-$C_6H_4$ | 2-F-$C_6H_4$ | $C_2H_5$ | N | | |
| 23 | 4-F-$C_6H_4$ | 4-F-$C_6H_4$ | $CH_3$ | N | | |
| 24 | 4-F-$C_6H_4$ | 4-F-$C_6H_4$ | $C_3H_7$ | N | | |
| 25 | 4-F-$C_6H_4$ | 2,4-$F_2$-$C_6H_3$ | $CH_3$ | N | | |
| 26 | 4-F-$C_6H_4$ | 2,6-$F_2$-$C_6H_3$ | $CH_3$ | N | | |
| 27 | 4-F-$C_6H_4$ | 2-$OCH_3$-$C_6H_4$ | $CH_3$ | N | | |
| 28 | 4-F-$C_6H_4$ | 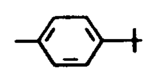 | $CH_3$ | N | | |

Tabelle: (Fortsetzung)

| Bsp. Nr. | $R^1$ | $R^2$ | $R^3$ | X | Schmp/IR | Isomer |
|---|---|---|---|---|---|---|
| 29 | $4-F-C_6H_4$ | $4-NO_2-C_6H_4$ | $CH_3$ | N | | |
| 30 | $4-F-C_6H_4$ | $4-NH_2-C_6H_4$ | $CH_3$ | N | | |
| 31 | $4-F-C_6H_4$ | $2-C_{10}H_7$ | $CH_3$ | N | | |
| 32 | $4-F-C_6H_4$ | Cyclohexyl | $CH_3$ | N | | |
| 33 | $C_6H_5$ | $2-Cl-C_6H_4$ | $CH_3$ | N | | |
| 34 | $C_6H_5$ | $2-Cl-C_6H_4$ | $CH_3$ | CH | | |
| 35 | $C_6H_5$ | $2-Cl-C_6H_4$ | $C_2H_5$ | N | | |
| 36 | $C_6H_5$ | $2-Cl-C_6H_4$ | $C_3H_7$ | N | | |
| 37 | $C_6H_5$ | $4-Cl-C_6H_4$ | $CH_3$ | N | | |
| 38 | $C_6H_5$ | $4-Cl-C_6H_4$ | $CH_3$ | CH | | |
| 39 | $C_6H_5$ | $2,4-Cl_2-C_6H_3$ | $CH_3$ | N | | |
| 40 | $C_6H_5$ | $2-F-C_6H_4$ | $CH_3$ | N | | |
| 41 | $C_6H_5$ | $4-F-C_6H_4$ | $CH_3$ | N | | |
| 42 | $C_6H_5$ | $2-CF_3-C_6H_4$ | $CH_3$ | N | | |
| 43 | $C_6H_5$ | $2-OCH_3-C_6H_4$ | $CH_3$ | N | | |
| 44 | $2-Cl-C_6H_4$ | $2-Cl-C_6H_4$ | $CH_3$ | N | | |
| 45 | $2-Cl-C_6H_4$ | $4-Cl-C_6H_4$ | $CH_3$ | N | | |
| 46 | $2-Cl-C_6H_4$ | $2-F-C_6H_4$ | $CH_3$ | N | | |

Tabelle: (Fortsetzung)

| Bsp. Nr. | $R^1$ | $R^2$ | $R^3$ | X | Schmp/IR | Isomer |
|---|---|---|---|---|---|---|
| 47 | 2-Cl-$C_6H_4$ | 4-F-$C_6H_4$ | $CH_3$ | N | | |
| 48 | 4-Cl-$C_6H_4$ | 2-Cl-$C_6H_4$ | $CH_3$ | N | | |
| 49 | 4-Cl-$C_6H_4$ | 2-Cl-$C_6H_4$ | $CH_3$ | CH | | |
| 50 | 4-Cl-$C_6H_4$ | 4-Cl-$C_6H_4$ | $CH_3$ | N | | |
| 51 | 4-Cl-$C_6H_4$ | 2-F-$C_6H_4$ | $CH_3$ | N | | |
| 52 | 4-Cl-$C_6H_4$ | 4-F-$C_6H_4$ | $CH_3$ | N | | |
| 53 | 4-Cl-$C_6H_4$ | 2-$CF_3$-$C_6H_4$ | $CH_3$ | N | | |
| 54 | 2,4-$Cl_2$-$C_6H_3$ | 2-Cl-$C_6H_4$ | $CH_3$ | N | | |
| 55 | 2,4-$Cl_2$-$C_6H_3$ | 2,4-$Cl_2$-$C_6H_3$ | $CH_3$ | N | | |
| 56 | 4-Br-$C_6H_4$ | 2-Cl-$C_6H_4$ | $CH_3$ | N | | |
| 57 | Cyclohexyl | 2-Cl-$C_6H_4$ | $CH_3$ | N | | |
| 58 | Cyclohexyl | 4-Cl-$C_6H_4$ | $CH_3$ | N | | |
| 59 | Cyclohexyl | 2-F-$C_6H_4$ | $CH_3$ | N | | |
| 60 | Cyclohexyl | 4-F-$C_6H_4$ | $CH_3$ | N | | |
| 61 | Cyclohexyl | 4-Cl-$C_6H_4$ | $C_2H_5$ | N | | |
| 62 | Cyclohexyl | 4-Cl-$C_6H_4$ | $C_3H_7$ | N | | |
| 63 | Cyclohexyl | 2,4-$Cl_2$-$C_6H_3$ | $CH_3$ | N | | |
| 64 | Cyclohexyl | 2,4-$Cl_2$-$C_6H_3$ | $CH_3$ | CH | | |

Tabelle: (Fortsetzung)

| Bsp. Nr. | $R^1$ | $R^2$ | $R^3$ | X | Schmp/IR | Isomer |
|---|---|---|---|---|---|---|
| 65 | 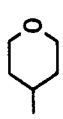 | 2-Cl-$C_6H_4$ | $CH_3$ | N | | |
| 66 | 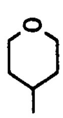 | 4-Cl-$C_6H_4$ | $CH_3$ | N | | |
| 67 | $CH_3$ | 2-Cl-$C_6H_4$ | $CH_3$ | N | | |
| 68 | $CH_3$ | 4-F-$C_6H_4$ | $CH_3$ | N | | |
| 69 | 4-F-$C_6H_4$ | 3-Pyridyl | $CH_3$ | N | | |
| 70 | 4-F-$C_6H_4$ | Norbornyl | $CH_3$ | N | | |
| 71 | 4-F-$C_6H_4$ | p-$C_{12}H_9$ | $CH_3$ | N | | |
| 72 | 4-F-$C_6H_4$ | 3-Cyclohexenyl | $CH_3$ | N | | |
| 73 | 4-F-$C_6H_4$ | 2-Cyclohexenyl | $CH_3$ | N | | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,

Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 2 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 2 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 1 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 2 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 1 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 2 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 1 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 2 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoff wurde cis-2-(1,2,4-Triazol-1-yl-methyl)-2-(tert.-butyl)-3(4-chlorphenyl)-oxiran (A) - bekannt aus DE-A 3 218 150 - benutzt.

Anwendungsbeispiel 1

Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95 %) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff und 20 % Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 und 22°C und 65 bis 70 % relativer Luftfeuchte aufgestellt. Nach 8 Tagen wurde das Ausmaß der Rostpilzentwicklung auf den Blättern ermittelt.

Das Ergebnis zeigt, daß die Wirkstoffe 1 und 2 bei der Anwendung als 0,006 gew.%ige Spritzbrühe eine bessere fungizide Wirkung (5 % Befall) zeigen als der bekannte Wirkstoff A (50 % Befall).

Anwendungsbeisplel 2

Wirksamkeit gegen Botrytis cinerea an Paprika

Paprikasämlinge der Sorte "Neusiedler Ideal Elite" wurden, nachdem sich 4 bis 5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten.

Das Ergebnis zeigt, daß die Wirkstoffe 1 und 2 bei der Anwendung als 0,05 gew.%ige Spritzbrühe eine bessere fungizide Wirkung (5 % Befall) zeigen als der bekannte Wirkstoff A (40 % Befall).

## Patentansprüche

**1.** 1-Alkoxy-1-azolylmethyloxirane der allgemeinen Formel I

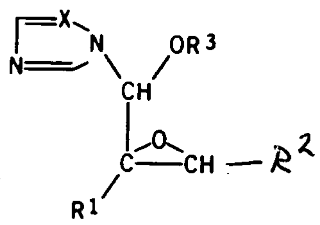

I,

in welcher

| | |
|---|---|
| $R^1$ und $R^2$ | gleich oder verschieden sind und jeweils $C_1$-$C_8$-Alkyl, $C_3$-$C_8$-Cycloalkyl, $C_5$-$C_8$-Cycloalkenyl, Tetrahydropyranyl, Norbornyl, Pyridyl, Naphthyl, Biphenyl oder Phenyl bedeuten, wobei diese Reste einfach bis dreifach durch Halogen, Nitro, Phenoxy, Amino, Alkyl, Alkoxy oder Halogenalkyl mit jeweils 1 bis 4 C-Atomen substituiert sein können, |
| $R^3$ | $C_1$-$C_4$-Alkyl bedeutet, |
| X | den Rest CH oder N bedeutet, |

sowie deren für Pflanzen verträgliche Säureadditionssalze und Metallkomplexe.

**2.** 1-Alkoxy-1-azolylmethyloxirane der allgemeinen Formel I, in der $R^1$ und $R^2$ die Phenylgruppe bedeuten, welche unsubstituiert oder durch einen oder zwei Substituenten substituiert ist, die Fluor, Chlor, Brom oder die Trifluormethylgruppe bedeuten.

**3.** Verfahren zur Herstellung der 1-Alkoxy-1-azolylmethyloxirane der Formel I gemäß Anspruch 1, dadurch gekennzeichnet, daß man eine Verbindung der Formel II

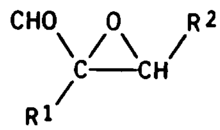

II,

in welcher $R^1$ und $R^2$ die angegebenen Bedeutungen haben, mit einer Verbindung der Formel III

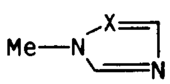

III,

in der Me ein Wasserstoffatom oder ein Metallatom bedeutet und X die oben angegebene Bedeutung hat, in Gegenwart von Thionylhalogeniden und Alkoholen umsetzt.

**4.** Fungizides Mittel, enthaltend einen festen oder flüssigen Trägerstoff und eine fungizid wirksame Menge eines 1-Alkoxy-1-azolylmethyloxiran der Formel I

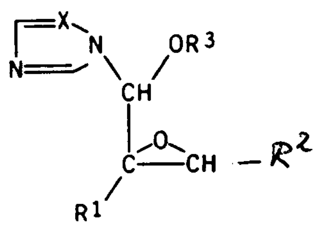

I,

in welcher

$R^1$ und $R^2$ gleich oder verschieden sind und jeweils $C_1$-$C_8$-Alkyl, $C_3$-$C_8$-Cycloalkyl, $C_5$-$C_8$-Cycloalkenyl, Tetrahydropyranyl, Norbornyl, Pyridyl, Naphthyl, Biphenyl oder Phenyl bedeuten, wobei diese Reste einfach bis dreifach durch Halogen, Nitro, Phenoxy, Amino, Alkyl, Alkoxy oder Halogenalkyl mit jeweils 1 bis 4 C-Atomen substituiert sein können,
$R^3$ $C_1$-$C_4$-Alkyl bedeutet,
X den Rest CH oder N bedeutet,
oder dessen für Pflanzen verträgliches Säureadditionssalz oder Metallkomplex.

**5.** Verfahren zur Bekämpfung von Pilzen, dadurch gekennzeichnet, daß man eine fungizid wirksame Menge eines 1-Alkoxy-1-azolylmethyloxirans der Formel I

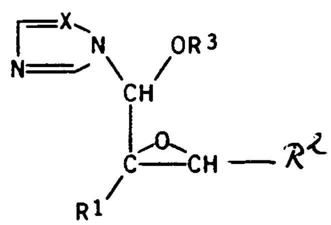

I,

in welcher
$R^1$ und $R^2$ gleich oder verschieden sind und jeweils $C_1$-$C_8$-Alkyl, $C_3$-$C_8$-Cycloalkyl, $C_5$-$C_8$-Cycloalkenyl, Tetrahydropyranyl, Norbornyl, Pyridyl, Naphthyl, Biphenyl oder Phenyl bedeuten, wobei diese Reste einfach bis dreifach durch Halogen, Nitro, Phenoxy, Amino, Alkyl, Alkoxy oder Halogenalkyl mit jeweils 1 bis 4 C-Atomen substituiert sein können,
$R^3$ $C_1$-$C_4$-Alkyl bedeutet,
X den Rest CH oder N bedeutet,
oder dessen für Pflanzen verträgliches Säureadditionssalz oder Metallkomplex auf die Pilze oder auf durch Pilzbefall bedrohte Materialien, Flächen, Pflanzen oder Saatgüter einwirken läßt.

**6.** Verbindung gemäß Anspruch 1, dadurch gekennzeichnet, daß $R^1$ 4-Fluorphenyl, $R^2$ Phenyl, $R^3$ Methyl und X N bedeuten.

**7.** Verbindung gemäß Anspruch 1, dadurch gekennzeichnet, daß $R^1$ 4-Fluorphenyl, $R^2$ Phenyl, $R^3$ Methyl und X CH bedeuten.

## Claims

**1.** A 1-alkoxy-1-azolylmethyloxirane of the general formula I

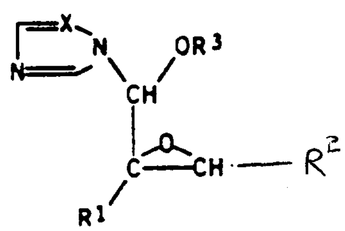

I,

where $R^1$ and $R^2$ are identical or different and each is $C_1$-$C_8$-alkyl, $C_3$-$C_8$-cycloalkyl, $C_5$-$C_8$-cycloalkenyl, tetrahydropyranyl, norbornyl, pyridyl, naphthyl, biphenylyl or phenyl, each of which can be substituted once to three times by halogen, nitro, phenoxy, amino, alkyl, alkoxy or haloalkyl of 1 to 4 carbon atoms in each case, $R^3$ is $C_1$-$C_4$-alkyl, X is CH or N, or an acid addition salt or metal complex thereof which is tolerated by plants.

**2.** A 1-alkoxy-1-azolylmethyloxirane of the general formula I where $R^1$ and $R^2$ are each phenyl which is unsubstituted or mono- or disubstituted by fluorine, chlorine, bromine or trifluoromethyl.

3. A process for the preparation of a 1-alkoxy-1-azolylmethyloxirane of the formula I as claimed in claim 1, which comprises reacting a compound of the formula II

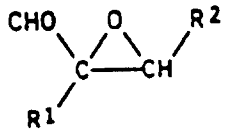

II,

where $R^1$ and $R^2$ have the stated meanings, with a compound of the formula III

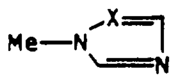

III,

where Me is hydrogen or a metal and X has the abovementioned meaning, in the presence of a thionyl halide and an alcohol.

4. A fungicidal agent containing a solid or liquid carrier and a fungicidally effective amount of a 1-alkoxy-1-azolylmethyloxirane of the formula I

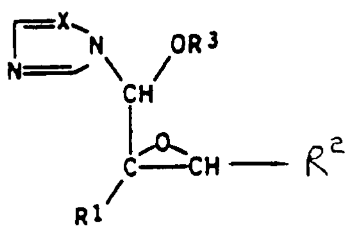

I,

where $R^1$ and $R^2$ are identical or different and each is $C_1$-$C_8$-alkyl, $C_3$-$C_8$-cycloalkyl, $C_5$-$C_8$-cycloalkenyl, tetrahydropyranyl, norbornyl, pyridyl, naphthyl, biphenylyl or phenyl, each of which can be substituted once to three times by halogen, nitro, phenoxy, amino, alkyl, alkoxy or haloalkyl of 1 to 4 carbon atoms in each case, $R^3$ is $C_1$-$C_4$-alkyl, X is CH or N, or an acid addition salt or metal complex thereof which is tolerated by plants.

5. A method for combating fungi, wherein a fungicidally effective amount of a 1-alkoxy-1-azolylmethyloxirane of the formula I

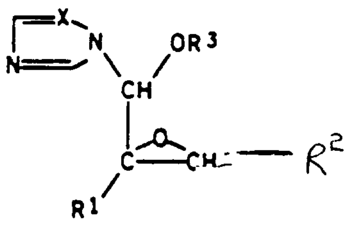

I,

where $R^1$ and $R^2$ are identical or different and each is $C_1$-$C_8$-alkyl, $C_3$-$C_8$-cycloalkyl, $C_5$-$C_8$-cycloalkenyl, tetrahydropyranyl, norbornyl, pyridyl, naphthyl, biphenylyl or phenyl, each of which can be substituted once to three times by halogen, nitro, phenoxy, amino, alkyl, alkoxy or haloalkyl of 1 to 4 carbon atoms in each case, $R^3$ is $C_1$-$C_4$-alkyl, X is CH or N, or an acid addition salt or metal complex thereof which is tolerated by plants, is allowed to act on the fungi or on the materials, areas, plants or seed threatened by fungal attack.

6. A compound as claimed in claim 1, wherein $R^1$ is 4-fluorophenyl, $R^2$ is phenyl, $R^3$ is methyl and X is N.

7. A compound as claimed in claim 1, wherein $R^1$ is 4-fluorophenyl, $R^2$ is phenyl, $R^3$ is methyl and X is CH.

**Revendications**

1. 1-alcoxy-1-azolylméthyloxirannes de formule générale I

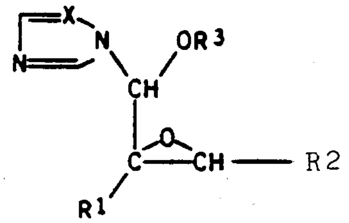

I,

dans laquelle
$R^1$ et $R^2$, ayant des significations identiques ou différentes, représentent chacun un groupe alkyle en $C_1$-$C_8$, cycloalkyle en $C_3$-$C_8$, cycloalcényle en $C_5$-$C_8$, tétrahydropyrannyle, norbornyle, pyridyle, naphtyle, biphényle ou phényle, ces radicaux pouvant porter 1 à 3 substituants halogéno, nitro, phénoxy, amino, alkyle, alcoxy ou halogénoalkyle contenant chacun 1 à 4 atomes de carbone,
$R^3$ représente un groupe alkyle en $C_1$-$C_4$,
X représente le groupe CH ou N,
et leurs sels formés par addition avec des acides et complexes métalliques tolérés par les végétaux.

2. 1-alcoxy-1-azolylméthyloxirannes de formule générale I dans laquelle $R^1$ et $R^2$ représentent des groupes phényle non substitués ou portant un ou deux substituants fluoro, chloro, bromo ou trifluorométhyle.

3. Procédé de préparation des 1-alcoxy-1-azolylméthyloxirannes de formule I selon la revendication 1, caractérisé en ce que l'on fait réagir un composé de formule II

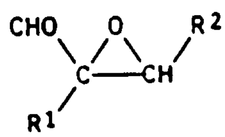

II,

dans laquelle $R^1$ et $R^2$ ont les significations indiquées avec un composé de formule III

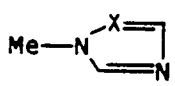

III,

dans laquelle Me représente un atome d'hydrogène ou de métal et X a les significations indiquées ci-dessus, en présence d'un halogénure de thionyle et d'alcools.

4. Produit fongicide contenant un véhicule solide ou liquide et une quantité fongicide efficace d'un 1-alcoxy-1-azolylméthyloxiranne de formule I

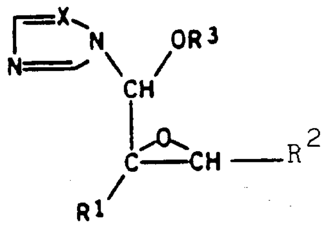

I,

dans laquelle
$R^1$ et $R^2$, ayant des significations identiques ou différentes, représentent chacun un groupe alkyle en $C_1$-$C_8$, cycloalkyle en $C_3$-$C_8$, cycloalcényle en $C_5$-$C_8$, tétrahydropyrannyle, norbornyle, pyridyle, naphtyle, biphényle ou phényle, ces radicaux pouvant porter un à trois substituants halogéno, nitro, phénoxy, amino, alkyle, alcoxy ou halogénoalkyle contenant chacun 1 à 4 atomes de carbone,
$R^3$ représente un groupe alkyle en $C_1$-$C_4$,
X représente le groupe CH ou N,
ou d'un sel formé par addition avec un acide ou d'un complexe métallique d'un tel composé, toléré par les végétaux.

**5.** Procédé pour combattre les mycètes, caractérisé en ce que l'on fait agir sur les mycètes ou sur les matériaux, surfaces, végétaux ou semences menacés d'un attaque par des mycètes une quantité fongicide efficace d'un 1-alcoxy-1-azolylméthyloxiranne de formule I

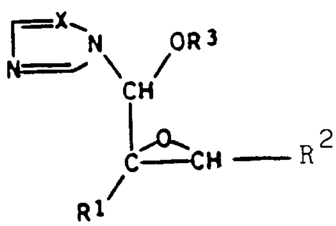

I,

dans laquelle
$R^1$ et $R^2$, ayant des significations identiques ou différentes, représentent chacun un groupe alkyle en $C_1$-$C_8$, cycloalkyle en $C_3$-$C_8$, cycloalcényle en $C_5$-$C_8$, tétrahydropyrannyle, norbornyle, pyridyle, naphtyle, biphényle ou phényle, ces radicaux pouvant porter 1 à 3 substituants halogéno, nitro, phénoxy, amino, alkyle, alcoxy ou halogénoalkyle contenant de 1 à 4 atomes de carbone,
$R^3$ représente un groupe alkyle en $C_1$-$C_4$,
X représente le groupe CH ou N,
ou d'un sel formé par addition avec un acide ou d'un complexe métallique d'un tel composé, toléré par les végétaux.

**6.** Composé selon la revendication 1, caractérisé en ce que $R^1$ représente un groupe 4-fluorophényle, $R^2$ un groupe phényle, $R^3$ un groupe méthyle et X représente N.

**7.** Composé selon la revendication 1, caractérisé en ce que $R^1$ représente un groupe 4-fluorophényle, $R^2$ un groupe phényle, $R^3$ un groupe méthyle et X représente CH.